Europäisches Patentamt

European Patent Office

Office européen des brevets

(11) Numéro de publication: **0 106 828 B1**

# FASCICULE DE BREVET EUROPEEN

(45) Date de publication de fascicule du brevet: **21.07.93**

(51) Int. Cl.5: **C12N 15/81**, C12P 21/02, A61K 39/29, C12N 1/18, C12N 1/20, //C12R1/19, C12R1/885

(21) Numéro de dépôt: **83870090.4**

(22) Date de dépôt: **06.09.83**

(54) **Molécules d'ADN recombinantes codant pour l'antigène de surface de l'hépatite B, leur préparation et vaccins qui en dérivent.**

(30) Priorité: **08.09.82 US 415789**

(43) Date de publication de la demande:
**25.04.84 Bulletin 84/17**

(45) Mention de la délivrance du brevet:
**21.07.93 Bulletin 93/29**

(84) Etats contractants désignés:
**AT BE CH DE FR GB IT LI LU NL SE**

(56) Documents cités:
**EP-A- 0 072 318**
**EP-A- 0 073 657**

**NATURE, vol. 298, no. 5872, 22 juillet 1982, pages 347-350, Macmillan Journals Ltd.; P. VALENZUELA et al.: "Synthesis and assembly of hepatitis B virus surface antigen particles in yeast"**

(73) Titulaire: **SMITHKLINE BIOLOGICALS S.A.**
**Rue du Tilleul, 13**
**B-1320 Genval Rixensart(BE)**

(72) Inventeur: **Cabezon, Theresa**
**rue de Terheyde, 78**
**B-1640 Rhode St Genèse(BE)**
Inventeur: **Crabeel, Marjolène**
**Dwersbos, 32**
**B-1630 Linkebeek(BE)**
Inventeur: **De Wilde, Michel**
**Avenue Gevaert, 136**
**B-1320 Rixensart (Genval)(BE)**
Inventeur: **Harford, Nigel**
**Jef Lambeaulaan, 9**
**B-1900 Overijse(BE)**

(74) Mandataire: **Tyrrell, Arthur William Russell et al**
**SmithKline Beecham, Corporate Patents, Great Burgh, Yew Tree Bottom Road Epsom, Surrey KT18 5XO (GB)**

CHEMICAL ABSTRACTS, vol. 95, no. 17, 26 octobre 1981, page 336, no. 147007c, Columbus, Ohio, US; M. CRABEEL et al.: "Cloning arg3, the gene for ornithine carbamoyltransferase from Saccharomyces cerevisiae: expression in Escherichia coli requires secondary mutations; production of plasmid beta-lactamase in yeast" & PROC. NATL. ACAD. SCI. U.S.A. 1981, 78(8), 5026-30

## Description

DOMAINE DE L'INVENTION

L'invention concerne des molécules d'ADN recombi-nantes codant pour l'antigène de surface de l'hépatite B, leur préparation et les vaccins qui en dérivent.

En d'autres termes, l'invention est relative au clonage d'un gène qui code pour l'antigène de surface de l'hépatite B dans la levure par application des techniques dites d'ADN recombinant ainsi qu'à la préparation d'un vaccin contre le virus de l'hépatite B à partir de l'antigène produit par la levure.

BASE DE L'INVENTION

L'infection par le virus de l'hépatite B (HBV) constitue un problème de santé grave et universel. L'infection peut se manifester en phase aigüe ou chronique. Le nombre de cas d'hépatite aigüe aux Etats-Unis est estimé à au moins 100.000 par année avec un taux de mortalité clinique de 1 à 2 %. Aux Etats-Unis, la prévalence des porteurs chroniques de HBV parmi les adultes sains varie entre 0,1 et 1 % selon l'âge et la classe sociale. En Amérique du Sud, la prévalence des porteurs chroniques est d'environ 1 à 3 %, en U.R.S.S. et en Europe méridionale, d'environ 3 à 6 % et en Asie et en Afrique, de plus de 10 %.

Dans les pays développés, les populations à haut risque d'exposition ont besoin d'un vaccin, notamment les malades et le personnel des unités médicales où on manipule le sang, le personnel militaire, les conjoints des porteurs chroniques, les voyageurs se déplaçant vers des zones de forte endémicité à HBV, les nouveauxnés des porteurs chroniques, les homosexuels, les prostituées et ceux qui font un mauvais usage des médicaments. Dans les pays du tiers-monde, il existe un besoin pour un vaccin bon marché destiné à l'immunisation des masses. Des programmes d'immunisation de masse peuvent influer en fin de compte seulement sur l'incidence de l'hépatite aigüe et celle du groupe des porteurs chroniques, mais ils peuvent également diminuer la morbidité et la mortalité provenant de l'hépatite chronique évolutive et du carcinome hépatocellulaire.

Les particules de Dane, que l'on croit être des virions de l'hépatite B et qui sont isolables à partir de malades contaminés, ont un diamètre d'environ 42 nm. Chaque particule se compose d'une enveloppe contenant l'antigène de surface de l'hépatite B (HBsAg), une capside (HBcAg), une polymérase endogène et un génome ADN. Le génome est circulaire et à double brin avec une région à simple brin comportant environ 200 bases. La région à simple brin peut être synthétisée in vitro par l'action de la polymérase endogène. Le brin le plus long contient environ 3.200 bases.

Il s'est avéré difficile de préparer des vaccins HBV parce qu'il s'est révélé malaisé de propager le virus dans une culture de tissu et parce que le seul hôte connu est l'homme. De petites quantités d'antigènes HBV authentiques ont été isolées à partir d'être humains contaminés. Les F-D-C Reports, pp 3-4, 19 juillet 1982, comportent un rapport d'études cliniques au sujet d'un vaccin développé récemment contre l'hépatite B.

Valenzuela et al., Nature, Volume 298, 347-350 (1982) mentionnent la synthèse de HBsAg dans la levure à l'aide d'un vecteur d'expression dans lequel la séquence codant pour HBsAg est un fragment TaqI-HpaI de 835 paires de bases (pb) et le promoteur est le promoteur de l'alcool-déshydrogénase I de la levure. Plusieurs rapports succincts antérieurs ont mentionné la recherche qui précède cette référence, à savoir, un rapport de Valenzuela et al., Arch. Biol. Med. Exp. (Chili) Volume 14(1), 21-22 (1981) qui mentionne l'expression dans la levure d'un fragment d'ADN contenant une séquence qui code pour une protéine analogue à HBsAg liguée à une région promoteur de l'alcool déshydrogénase de la levure; un rapport dans Scrip. N° 616 p 14 (12 août 1981) qui relate qu'une équipe de chercheurs des Etats-Unis comprenant P. Valenzuela et W.J. Rutter a annoncé la production dans la levure de l'"enveloppe protéinique entourant le virus de l'hépatite B"; et un rapport de Zuckerman, Nature, Volume 295, 98-99 (1982) qui mentionne que W.J. Rutter a signalé l'expression de HBsAg glycosylé dans des cellules de levure.

Des composants antigéniques de HBV, tels que HBsAg, ont été préparés dans des bactéries après l'insertion d'une molécule d'ADN recombinante contenant un gène qui code pour l'antigène. Burrell et al., Nature, Volume 279 N° 5708, 43-47 (1979) mentionnent l'expression dans la souche HB 101 de E. coli des séquences d'ADN de HBV clonées dans le plasmide pBR 322.

Murray et al., demande de brevet européen N° 12828, décrivent la préparation d'un vecteur recombinant qui peut coder pour les antigènes HBV, y compris HBsAg, dans la souche HB 101 de E. coli. Le vecteur est préparé à partir de l'ADN de la particule de Dane et du plasmide pBr 322. Les auteurs énoncent que les hôtes utiles peuvent englober d'autres hôtes bactériens, des levures et d'autres champignons, des cellules animales ou végétales et d'autres hôtes, bien que le seul hôte démontré soit le E. coli.

Charnay et al., Nature, Volume 286, 893-895 (1980) mentionnent la construction d'un bactériophage comportant une fusion de gène de la β-galactosidase et du gène de structure de l'HBsAg. Le bactériophage dirige la synthèse d'une protéine de fusion comprenant les déterminants antigéniques de l'HBsAg et de la β-galactosidase.

Tiollais et al., demande de brevet du Royaume-Uni N° 2034323, décrivent la préparation d'un coliphage contenant l'ADN de HBV. L'ADN de HBV fusionné à l'ADN du phage est transformé dans la souche C600 de E. coli.

Dans la demande de brevet du Royaume Uni N° 2070621, publiée d'abord sous forme de demande PCT W081/00577, on décrit un plasmide qui comprend une partie du gène de l'HBsAg et le promoteur et le gène Z de l'opéron lactose et qui peut être cloné dans E. coli.

Rutter et al., demande de brevet européen N° 20251, décrivent des vecteurs recombinants comprenant un vecteur recombinant qui contient le plasmide pBR322 et des fragments BamHI de l'ADN de HBV qui peuvent être utilisés pour transformer le E. coli. Un autre vecteur comprenant un fragment BamHI de l'ADN de HBV et une portion de l'opéron tryptophane, a été utilisé pour obtenir l'expression dans la souche de E. coli HB101.

Edman et al., Nature, Volume 291, Numéro 5815, 503-506 (1981), décrivent la construction de plasmides qui dirigent la synthèse de HBcAg et de la protéine de fusion β-lactamase-HBsAg, sous le contrôle de la région de régulation de l'opéron tryptophane, dans E. coli.

D'autres références décrivant l'insertion de l'ADN de HBV dans des bactéries englobent celles de Charnay et al., Prog. Med. Virol., Volume 27, 88-92 (1981); Mac Kay et al., Proc. Natl. Acad. Sci. U.S., Volume 78, Numéro 7, 4510-4514 (1981); Fritsch et al., C.R. Acad. Sci., Volume 287, Numéro 16, 1453 (1978); Brevet du Royaume-Uni N° 2034323 (Derwent N° 46874C) et Pasek et al., Nature, Volume 282 N° 6, 575 (1979).

L'ADN de HBV a également été cloné dans des cellules de mammifères. Celles-ci comprennent les lignées cellulaires d'origine humaine, celles de souris et celles de l'hépatome humain. Par exemple, Dubois et al., Proc. Natl. Acad. Sci. U.S., Volume 77, Numéro 8, 4549-4553 (1980), mentionnent la transformation des cellules de souris à l'aide de plasmide contenant le génome HBV et l'expression de HBsAg; Hirschman et al., Proc. Natl. Acad. Sci. U.S., Volume 77, Numéro 9, 5507-5511 (1980) mentionnent la production de particules ressemblant à HBV par des cellules HeLa transformées avec l'ADN de HBV.

Des procédés de préparation du vaccin HBV en utilisant HBsAg à partir du sang humain sont mentionnés par Funakoshi et al., Prog. Med. Virol., Volume 27, 163-167 (1981) et Maupas et al., Prog. Med. Virol., Volume 27, 185-201 (1981). Le vaccin préparé par Funakoshi et al., contient 40 μg de HBsAg traité par le formol et purifié, du phosphate/chlorure de sodium, 20 mg de mannitol et 0,1 % d'hydroxyde d'aluminium comme adjuvant. Dans le dernier document, Maupas et al. mentionnent qu'une dose de vaccin représentait 1 ml de HBsAg traité par le formol et purifié contenant 2-10 μg/ml de protéine (méthode de Lowry) et 0,1 % d'hydroxyde d'aluminium. Le protocole utilisé dans l'étude mentionnée par Maupas et al., impliquait trois injections à intervalles d'un mois avec un rappel après un an; les auteurs proposent un protocole consistant en deux injections de HBsAg concentré à intervalles de trois mois.

Des références supplémentaires concernant la préparation des vaccins HBV englobent celles de Maupas et al., respectivement aux pages 3, 37, et 57 de Hepatitis B Vaccine INSERM Symposium N° 18, édité par Maupas et Guesry, 1981, Elsevier/North Holland Biomedical Press.

Des levures ont été utilisées comme organismes hôtes pour certaines autres séquences d'ADN. Par exemple, Fraser et al., demande de brevet du Royaume-Uni n° 2068969, décrivent la préparation de l'ovalbumine de poulet dans la levure; Scrip N° 640, p. 11 (4 novembre 1981) contient un rapport selon lequel un type d'interféron serait préparé dans la levure. Dans le brevet européen N° 11562 (Derwent N° 38762C), on décrit des plasmides hybrides de levure contenant le gène $ura_3^+$ de levure dans le plasmide 2 μ.

## RESUME DE L'INVENTION

L'invention est relative à la préparation d'une molécule d'ADN recombinante comprenant une séquence nucléotidique qui peut coder pour HBsAg et une région de régulation provenant du gène arg3 de la levure qui peut effectuer la transcription de la séquence de HBsAg dans la levure Saccharomyces cerevisiae. La molécule comprend des vecteurs dans lesquels la séquence HBsAg et la région de régulation ont été insérées, lesquels vecteurs peuvent être utilisés pour préparer un vecteur de levure ou pour conserver l'HBsAg et les régions de régulation. Les microorganismes contenant la molécule d'ADN recombinante, notamment les microorganismes transformés par les plasmides de l'invention, font partie de l'invention.

L'invention comprend également un vaccin destiné à stimuler la protection contre l'infection à HBV chez les humains et qui contient une quantité vaccinale de HBsAg préparée suivant la présente invention ainsi qu'un excipient convenable.

En outre, l'invention comprend des procédés pour préparer la molécule d'ADN recombinante et les microorganismes contenant les molécules ainsi que des procédés pour préparer l'HBsAg et le vaccin contenant l'HBsAg.

## BREVE DESCRIPTION DES FIGURES

La figure 1 représente une carte de clivage du pRIT10601 par une série d'endonucléases de restriction.

La figure 2 représente une carte de clivage du pRIT10616 par endonucléase de restriction.

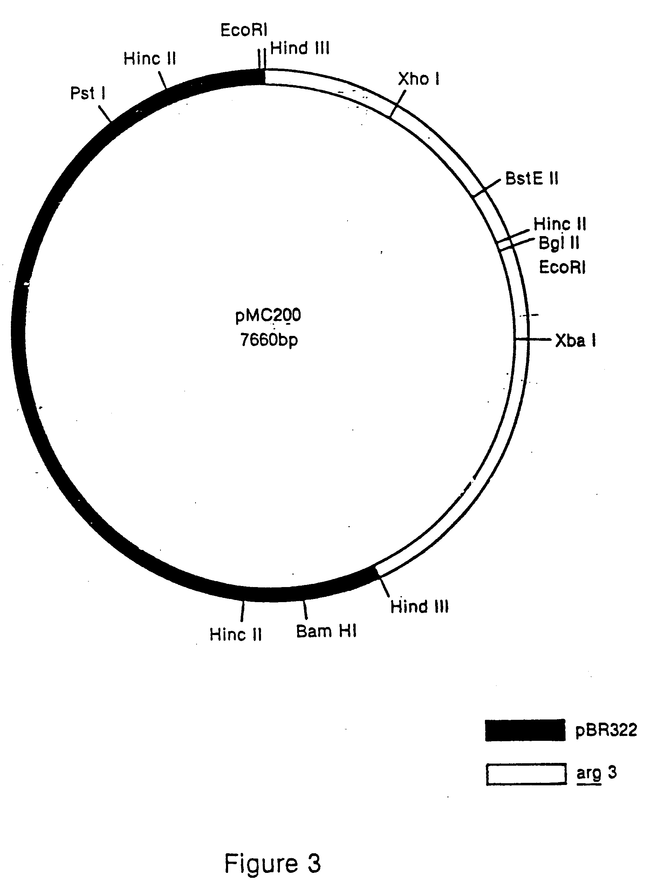

La figure 3 représente une carte de clivage du pMC200 par endonucléase de restriction.

La figure 4 représente la séquence nucléotidique d'une portion de l'ADN de levure inséré sous forme d'un fragment HindIII de 3300 pb dans pMC200 laquelle portion contient les sites HincII, BlgII et EcoRI.

La figure 5 représente un diagramme illustrant la préparation du pRIT01671 et du pRIT10673.

## DESCRIPTION DETAILLEE DE L'INVENTION

La molécule d'ADN recombinante de l'invention est préparée par fusion d'une séquence nucléotidique qui comprend un gène de structure pour HBsAg avec la région de régulation arg3 de levure, laquelle région de régulation peut diriger la transcription de la séquence de HBsAb dans la levure en réalisant ainsi son expression. Par "molécule d'ADN recombinante", on entend un fragment d'ADN qui contient la séquence codant pour HBsAg et la région de régulation ainsi que d'autres molécules d'ADN contenant le fragment, telles que plasmides ou phages vecteurs.

Par "région de régulation", on entend une séquence qui contient une région promoteur et d'autres séquences nécessaires pour la transcription. La région de régulation arg3 de levure est particulièrement avantageuse parce qu'elle peut être un promoteur puissant pour l'expression d'une séquence codant pour HBsAg.

Par "HBsAg", on entend une protéine qui est structurellement identique à l'authentique HBsAg ou qui possède effectivement les mêmes déterminants antigéniques que l'authentique HBsAg, c'est-à-dire une protéine capable de stimuler la production d'anticorps qui reconnaissent spécifiquement l'HBsAg authentique et réagissent avec lui ou une protéine spécifiquement reconnue par les anticorps anti-HBsAg.

La séquence codant pour HbsAg peut être isolée de l'ADN extrait des particules de Dane dans le sérum humain en synthétisant la fibre complémentaire à la région simple brin à l'aide d'une ADN polymérase, de préférence la polymérase endogène, suivi d'une digestion à l'aide d'une endonucléase de restriction. Le choix de l'endonucléase dépendra en partie des particules de Dane. Par exemple, ainsi qu'il est illustré dans les exemples ci-dessous, la séquence codant pour HbsAg de l'ADN de HBV de certaines particules de Dane de sérotype adw peut être isolée sur un simple fragment BamHI; la séquence codant pour HBsAg de l'ADN de HBV de certaines particules de Dane de sérotype ayw peut être isolée sur un fragment HhaI. L'ADN isolé des particules de Dane de même sérotype peut également présenter différents motifs de sites de restriction.

La restriction de l'ADN en vue de préparer les fragments d'ADN utilisés dans l'invention, la ligation de tels fragments pour préparer les molécules d'ADN recombinantes utilisées dans l'invention et l'insertion dans des microorganismes sont réalisées selon des techniques connues telles que les techniques décrites dans les références citées précédemment et ultérieurement. On choisit les conditions de façon à éviter la dénaturation de l'ADN et celle des enzymes. Par exemple, on tamponne le pH de façon à le maintenir entre 7 et 11 et on maintient la température en dessous de 60° C. On effectue la restriction de préférence à une température d'environ 30 à 40° C et on effectue la ligation à environ 0 à 10° C. Les enzymes de restriction et les ligases utilisées au cours de la réalisation de la présente invention sont disponibles commercialement et devraient être utilisées conformément aux directives qui y sont incluses. L'ADN ligase de T4 est la ligase préférée.

La fusion de la séquence de HBsAg à la région de régulation peut s'effectuer à l'aide de vecteurs intermédiaires, ainsi qu'il est illustré dans les exemples ci-dessous. La séquence de HBsAg peut encore être insérée directement dans un vecteur qui contient la région de régulation. Ce vecteur est l'ADN qui peut transporter et conserver le fragment d'ADN de l'invention y compris, par exemple, les phages et les plasmides. Les techniques pour cloner les fragments d'ADN dans les phages sont décrites, par exemple, par Charnay et al., Nature, Volume 286, 893-895 (1980) et Tiollais, demande de brevet du Royaume-Uni n° 2034323. De préférence, la séquence HBsAg est positionnée par rapport à la région de régulation de façon

5

que l'HbsAg synthétisé par expression de la séquence de HBsAg est pratiquement dépourvu de résidus en acides aminés étrangers.

Une région de régulation que l'on a découvert être particulièrement utile provient du gène arg3 de levure qui code pour l'ornithine carbamoyltransférase (OCT). L'utilisation de la région de contrôle arg3 est avantageux parce que son activité est soumise à la fois à des mécanismes de contrôle spécifiques et généraux. On l'a clonée dans E. coli sur le plasmide pYeura3arg3 ainsi qu'il est signalé par Crabeel et al., Proc. Natl. Acad. Sci. U.S.A., Volume 78, 5026 (1981). Les hôtes préférés sont les souches de S. cerevisiae, notamment la souche lc11697d, dans lesquelles la voie d'accès biosynthétique de l'arginine est déréprimée. L'emploi de telles souches entraîne une expression accrue à partir du promoteur arg3 comparée à l'autre-souche, la souche DC5, qui a été utilisée dans les exemples.

Le vecteur préféré pour cloner le fragment d'ADN fusionné dans la levure est le plasmide YEp13, qui est capable de réplication et de conservation à la fois dans E. coli et S. cerevisiae et est par conséquent connu sous le nom de vecteur "navette". Plusieurs autres vecteurs de levure sont connus et disponibles. L'HBsAg et les régions de régulation peuvent être insérés séquentiellement dans un vecteur de levure ou, ainsi qu'il est illustré dans les exemples ci-dessous, simultanément. La transformation par des vecteurs plasmidiques entraînera normalement l'incorporation de la molécule d'ADN de l'invention sous forme d'un plasmide. Cependant, d'autres réactions, telles que des événements de recombinaison, peuvent entraîner l'incorporation de la molécule d'ADN dans l'ADN chromosomique.

Les vaccins destinés à stimuler la protection contre l'infection à HBV chez les humains et qui comprennent l'HBsAg produit par la levure suivant l'invention et un excipient convenable peuvent être préparés par des techniques connues. L'emploi d'un adjuvant tel que l'hydroxyde d'aluminium est souhaitable. L'HBsAg ainsi produit peut également être combiné à d'autres antigènez pour préparer des combinaisons de vaccins. Les vaccins HBV et les combinaisons de vaccins peuvent s'administrer, par exemple, par voie sous-cutanée, intraveineuse ou intramusculaire. En faisant appel aux techniques d'hybridation d'ADN et aux diverses analyses immunologiques, on peut également utiliser comme sonde le fragment d'ADN de l'invention et l'HBsAg ainsi produit pour détecter HBV dans des échantillons biologiques.

EXEMPLES

Dans les exemples de l'invention qui suivent et qui sont illustratifs mais non limitatifs, tous les pourcentages figurent en poids et toutes les températures figurent en degrés Celsius.

Exemple 1. Préparation du plasmide intermédiaire pRIT10601 par combinaison de l'ADN de HBV avec pBR322.

On a défibriné du sérum positif de l'HBsAg de sérotype ayw par addition de $CaCl_2$ jusqu'à une concentration finale de 0,28 % et on l'a centrifugé pendant 2 heures à 27.000 tpm dans un rotor SW27 sur gradients de saccharose à 10-20 % préparé dans un tampon (pH 7,5) contenant 10 mM de trométhamine-HCl, 1 M de NaCl et 1 mM de EDTA. On a remis en suspension dans le même tampon un culot transparent contenant les particules de Dane et on l'a centrifugé sur du saccharose tamponné à 20 % disposé en couches sur un coussin de saccharose à 65 %. On a récupéré une bande opalescente à l'interface du coussin et on l'a centrifugé pendant 4 heures à 200.000 G pour culotter les particules de Dane.

A. On a réparé les régions simple brin du génome HBV à l'intérieur des particules de Dane par l'ADN polymérase endogène en remettant les particules de Dane en suspension dans un mélange réactionnel (pH 8) contenant 50 mM de dithiothréitol, 50 mM de chacun des dATP, dCTP et dGTP et 8 $\mu$M de 32p-dTTP (350 Ci/mmole) et en incubant les particules remises en suspension pendant 5 heures à 37° C. La resuspension à pH 7,5 à l'aide d'un tampon contenant 10 mM de trométhamine-HCl, 10 mM de EDTA, 100 M de NaCl et du dodécyl sulfate de sodium à 0,02 % (pH 7,5) et on l'a incubé pendant 1 heure à 37° C avec 0,5 mg/ml de pronase suivi d'une extraction au phénol et d'une précipitation à l'éthanol.

La digestion de l'ADN par l'endonucléase de restriction BamHI a fourni deux fragments radioactifs ayant des dimentions d'environ 1450 pb et 2600 pb ainsi qu'il a été estimé par électrophorèse sur gel d'agarose et par autoradiographie du gel.

B. On a réparé environ 30 ng d'ADN de particule de Dane avec l'ADN polymérase endogène en présence de dTTP non marqué, on les a extraits et on les a récupérés comme il est décrit ci-dessus. On a mélangé l'ADN avec 100 ng de plasmide pBR322 qui a été préalablement digéré par l'endonucléase de restriction BamHI et traité par la phosphatase alcaline. Le plasmide pBR322 est généralement utilisé dans les techniques dites d'ADN recombinant et se trouve en dépôt et accessible sans restriction à l'American Type Culture Collection sous le numéro d'ordre 37017. On a extrait le mélange au phénol, on l'a précipité par l'éthanol, on l'a centrifugé, on l'a séché et on l'a remis en suspension dans 12 ml d'un mélange (pH 7,5) contenant 50 mM de trométhamine-HCl, 1 mM d'ATP, 10 mM de MgCl$_2$, 10 mM de dithiothréitol, 50 ug/ml de gélatine et 2 unités/ml d'ADN ligase de T4. On a incubé la suspension à 10° pendant 4 h. et on l'a maintenue ensuite sur de la glace pendant 18 h.

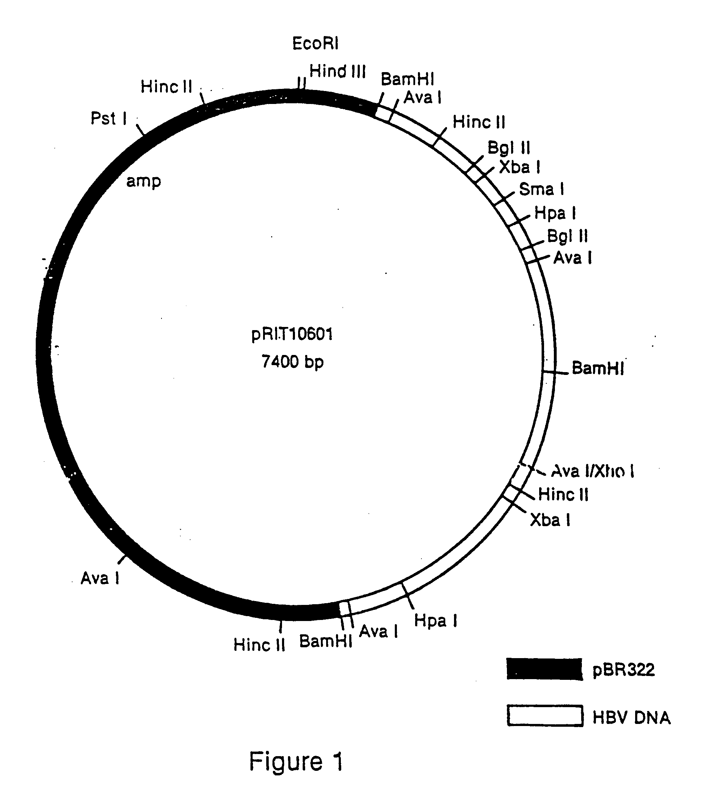

On a utilisé le mélange d'ADN ligué pour transformer les cellules compétentes de la souche C600 de E. coli K12 préparées suivant le procédé de Cohen et al., Proc. Natl. Acad. Sci. U.S., Volume 69, 2110 (1972). On a sélectionné les transformants sur un milieu solide contenant de l'ampicilline (200 μg/ml). On a criblé les colonies isolées en fonction de la perte de résistance à la tétracycline, ce qui a dénoté l'insertion d'un fragment d'ADN étranger dans le site BamHI du pBR322. On a constaté qu'un tel clone transformant contient un plasmide pRIT10601, lequel, par digestion avec l'endonucléase BamHI, a fourni un fragment pBR322 de 4360 pb et des fragments d'ADN de HBV de 1600 pb et de 1450 pb. Une culture de la souche C600 de E. coli K12 (pRIT10601) a été déposée le 2 juin 1982, conformément aux dispositions de la Convention sur le brevet européen (CBE) et du Traité de Budapest, à l'American Type Culture Collection, Rockville, Maryland, E.U.A., sous le numéro d'ordre ATCC 39132. Une carte de clivage du plasmide par l'endonucléase de restriction pRIT10601 est donnée à la figure 1.

C. Les dimensions des fragments générés par digestion de l'ADN de la particule de Dane et du pRIT10601 avec divers enzymes de restriction ont été comparées comme suit. On a marqué l'ADN de la particule de Dane, c'est-à-dire l'ADN de HBV, avec du $^{32}$P par la réaction à la polymérase endogène décrite ci-dessus ou en traitant l'ADN purifié avec l'ADN polymérase I en provenance de E. coli. On a mélangé l'ADN de HBV marqué avec le pRIT10601, on a traité le mélange par une endonucléase de restriction et on a procédé à l'électrophorèse sur un gel d'agarose. On a coloré le gel à l'aide de bromure d'éthidium et on l'a photographié sous rayonnement UV afin de localiser les fragments d'ADN, on l'a ensuite séché et on l'a autoradiographié afin de localiser les fragments d'ADN de HBV radioactifs. On a trouvé que les fragments suivants de HBV correspondaient exactement à la dimension des fragments du pRIT 10601 : les fragments BamHI de 1450 pb et de 1600 pb; un fragment HpaI de 1330 pb et un fragment BamHI-XhOI de 1130 pb. L'ADN marqué de la particule de Dane s'hybridait également spécifiquement avec les deux fragments de 1450 pb et 1600 pb libérés par la digestion avec BamHI du pRIT10601 en suivant le transfert de ces fragments à partir d'un gel d'agarose sur un filtre de nitrocellulose selon la technique de Southern J. Mol. Biol., Volume 98, 503 (1975).

Ces résultats montrent que l'insertion d'ADN cloné sur pRIT10601 représente le génome HBV et que l'orientation relative des deux fragments BamHI sur pRIT10601 est la même que dans le virion.

On a utilisé pRIT10601 pour préparer pRIT10671 dans l'exemple 10 ci-dessous.

Exemple 2. Préparation du plasmide intermédiaire pRIT10601 par combinaison de l'ADN de HBV avec pACYC184.

On a isolé les particules de Dane en provenance du sérum positif de HBsAg de sérotype adw, comme il a été décrit ci-dessus. L'analyse par endonucléase de restriction de l'ADN de HBV marqué au $^{32}$P indiquait que l'ADN contenait un site EcoRI.

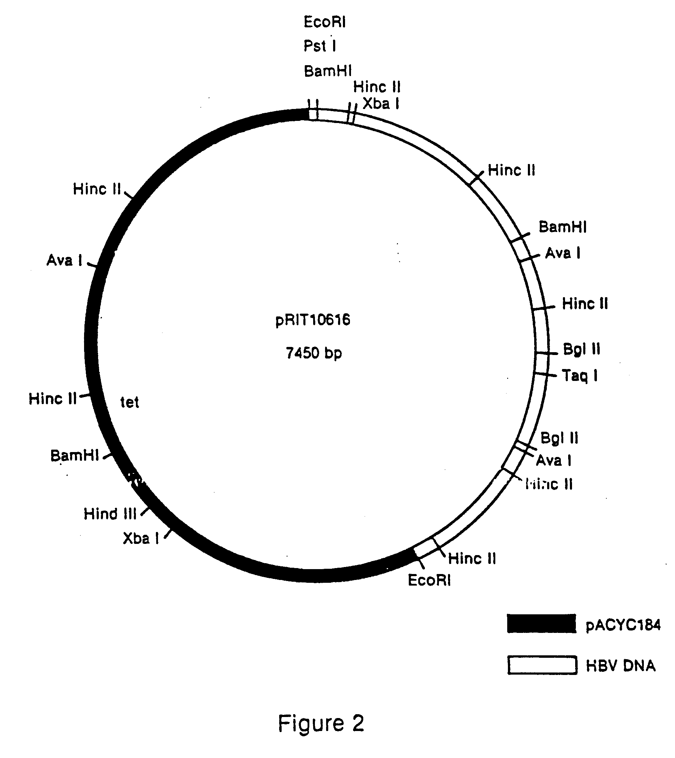

On a fait digérer par EcoRI, l'ADN de HBV complété par la réaction à la polymérase endogène à l'aide de nucléotides non marqués. On a mélangé l'ADN digéré avec le plasmide pACYC184 qui avait été préalablement digéré par EcoRI et traité par la phosphatase alcaline. Le plasmide pACYC184 se trouve en dépôt et accessible sans restriction, à l'American Type Culture Collection, sous le numéro d'ordre 37033. On a ligué le mélange d'ADN ligase de T4. On a utilisé le mélange d'ADN ligué pour transformer les cellules compétentes de la souche C600 de E. coli. On a sélectionné les transformants sur un milieu tétracycline (15 ug/ml) et on les a criblés en fonction de la perte de résistance au chloramphénicol, ce qui a dénoté l'insertion du pACYC184 dans le site EcoRI. On a trouvé qu'une colonie transformée contient un plasmide pRIT10616, lequel se compose du pACYC184 muni d'un insert de 3200 pb comprenant l'ADN de HBV au site EcoRI. Une carte de restriction du pRIT10616 est donnée à la figure 2. La souche C600 de E. coli K12 (pRIT 10616) a été déposée le 2 juin 1982, conformément aux dispositions de la CBE et du Traité de Budapest, à l'American Type Culture Collection sous le numéro d'ordre ATCC 39131.

Exemple 3. Préparation du plasmide intermédiaire, pRIT10640, contenant une séquence nucléotidique codant pour HBsAG, par combinaison du pRIT10616 avec pBR313.

On a purifié le pRIT10616 par centrifugation en gradient de densité d'un mélange CsCl/bromure d'éthidium, en substance, comme décrit par Kahn et al., Methods in enzymology, Volume 68, 268 (1979).

On a fait digérer l'ADN par l'endonucléase BamHI, on l'a mélangé avec l'ADN du plasmide pBR313 digéré par BamHI et traité par la phosphatase alcaline, on l'a ligué et on l'a utilisé pour transformer les cellules compétentes de la souche C600 de E. coli K12 en substance, comme décrit dans l'exemple 1. Le plasmide pBR 313 se trouve en dépôt et accessible sans restriction à l'American Type Culture Collection sous le numéro d'ordre 37018.

On a sélectionné les transformants sur de la gélose contenant de l'ampicilline et on les a criblés en fonction de la perte de résistance à la tétracycline ce qui a dénoté l'insertion du pBR313 au site BamHI. On a trouvé qu'une colonie transformée contenait un plasmide pRIT10640, lequel se compose du pBR313 muni d'un insert de 1350 pb au site BamHI. L'insert est une séquence nucléotidique qui peut coder pour HbsAg. Il code pour la partie d'une protéine précurseur possible de HbsAg et l'antigène de surface total comporte 565 pb des séquences non codantes 3'.

Exemple 4. Préparation du plasmide intermédiaire pMC200, contenant la région de régulation arg3 par combinaison du gène arg3 de levure avec pBR322.

On a obtenu un fragment d'ADN de levure de 3300 pb contenant le gène arg3 par digestion du pYeura3arg3 avec HindIII. Le plasmide pYeura3arg3 a été décrit par Crabeel et al., Proc. Natl. Acad. Sci. U.S. Volume 78, 5026 (1981). On a cloné le fragment de 3300 pb dans le site HindIII du pBR322 et on l'a transformé en la souche MM294 de E. coli K12, en substance, comme il a été décrit ci-dessus. On a sélectionné les transformants sur milieu d'ampicilline et on les a criblés en fonction de la résistance à la tétracycline. On a trouvé qu'une colonie transformée contenait un plasmide pMC200 lequel comprend pBR322 muni d'un insert au site HindIII. Ce plasmide contient la région de régulation arg3 qui peut effectuer la transcription d'une séquence nucléotidique de HBsAg dans les cellules de levure comme il a été décrit dans les exemples suivants. La souche MM294 de E. coli K12 a été déposée le 2 juin 1982 conformément aux dispositions de la CBE et du Traité de Budapest, à l'American Type Culture Collection, sous le numéro d'ordre ATCC 39130.

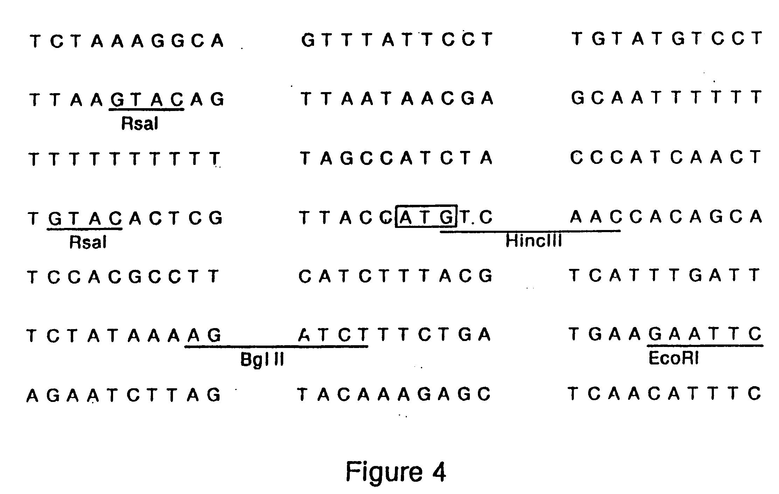

La séquence nucléotidique de la partie du gène arg3, comprenant la partie des séquences N-terminales codantes pour l'ornithine carbamoyltransférase (OCT) et la partie de la région 5' non traduite a été déterminée par Huyghen et al., Arch. Intl. Physical. Biochem., Volume 89, B172 (1981). La figure 4 illustre un fragment de 210 pb de la séquence connue. Le fragment de 210 pb contient des sites HincII, BglII et EcoRI uniques sur l'ADN de levure insérée sous forme de HindIII de 3300 pb dans pMC200. On pense que le codon d'initiation pour la séquence codante pour la protéine OCT correspond au codon ATG encadré. On suppose que l'introduction des séquences codantes pour HbsAg seul ou le précurseur de HbsAg plus HbsAg dérivé de l'ADN de HBV cloné dans les sites HincII, BglII ou EcoRI de l'ADN de levure se traduit par une fusion de gène et la production d'une protéine hybride transcrite et traduite à partir de la région de régulation arg3 à condition que la fusion du gène soit effectuée dans l'orientation correcte et que le cadre de lecture soit maintenu pour la traduction au-delà du site de fusion.

Exemple 5. Préparation des plasmides intermédiaires pRIT10671 et pRIT10672, contenant HbsAg et les régions de régulation par combinaison du pRIT10641 avec le pMC200.

On fait digérer 5 μg de pMC200 avec 6,4 unités de BglII pendant 2,5 h. à 37°, on les a dilués avec un volume égal d'un tampon (pH 10,5) contenant 0,1 M de glycine, 0,01 M de MgCl$_2$6H$_2$O et 0,1 mil de ZnCl$_2$ et on les a incubés avec 0,5 unité de phosphatase alcaline d'intestin de veau pendant 30 minutes à 37° de façon à éliminer les résidus phosphates 5' terminaux. On a extrait le mélange à deux reprises avec du phénol saturé en tampon et trois fois avec de l'éther. On a précipité l'ADN avec de l'éthanol et on l'a dissous dans 0,01 M d'un tampon trométhamine.

On a fait digérer 25 μg de pRIT10640 par l'endonucléase BamHI afin de couper le fragment BamHI de 1350 pb par électrophorèse préparatoire sur gel d'agarose et par électroélution. On a récupéré l'ADN élué et on l'a concentré par précipitation à l'éthanol et on l'a dissous dans 20 μl de tampon trométhamine 0,01 M (pH 7). On a mélangé une aliquote du fragment BamHI (0,3 ug) qui contient la séquence codant pour HBsAg avec 0,5 ug du pMC200 digéré par BglII et on a ligué le mélange par incubation avec l'ADN ligase de T4.

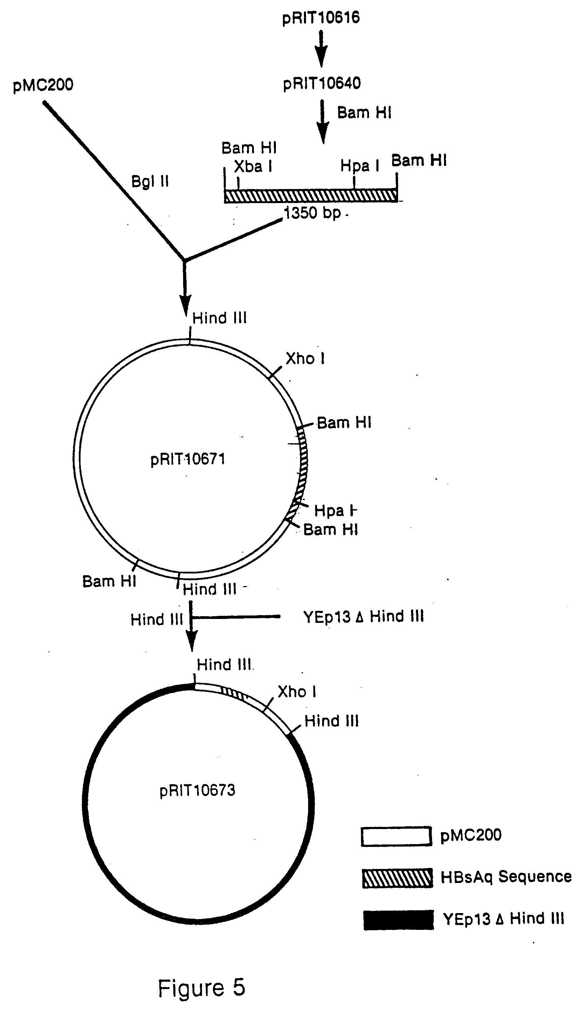

On a utilisé l'ADN ligué pour transformer les cellules compétentes de la souche MM294 de E. coli K12. On a sélectionné les transformants sur des plaques de gélose contenant 200 μg/ml d'ampicilline. On a isolé douze colonies résistantes par passages successifs sur gélose à base d'ampicilline et on a isolé les plasmides selon le procédé décrit par Birnboim et al., Nucl. Acid. Res. Volume 7, 1513 (1979). L'analyse par électrophorèse en gel d'agarose a montré que tous les plasmides ont été digérés par HpaI, ce qui dénote l'insertion du fragment BamHI et que les deux orientations du fragment inséré étaient présentes parmi les douze colonies transformées. On a isolé les plasmides par centrifugation en gradient de densité d'un mélange CsCl/ bromure d'éthidium. Un plasmide, pRIT10671, contient le fragment BamHI fusionné au site BglII dans l'orientation correcte pour la transcription de la séquence codant pour HbsAg de façon à engendrer une protéine de fusion de 286 acides aminés comprenant 18 acides aminés N-terminaux de OCT, 42 acides aminés de la protéine précurseur possible de HBsAg et les 226 acides aminés de HBsAg. La protéine de fusion est HBsAg comme indiqué dans l'exemple 8 ci-dessus. Les transformants contenant pRIT10671 sont appelés souche MM294 de E. coli K12 (pRIT10671). pRIT10671 est illustré dans la figure 5.

L'utilisation du pRIT10640 comme plasmide intermédiaire n'est pas indispensable. Par exemple, le fragment BamHI pourrait avoir été excisé à partir de pRIT10616.

Un autre plasmide, le pRIT10672, contenant le fragment BamHI dans la disposition incorrecte pour la transcription de la séquence codant pour HBsAg. Les transformants contenant ce plasmide sont appelés souche MM294 de E. coli K12 (pRIT10672).

Exemple 5. Préparation des plasmides, les vecteurs "navette" pRIT10673 et pRIT10674, par combinaison des pRIT10671 et pRIT10672 avec le vecteur "navette" YEp13.

Le vecteur YEp13 est un vecteur "navette" entre S. cerevisiae et E. coli. Il a été décrit par Broach et al. Gene, Volume 8, 121 (1979). Il est fourni par J. Hicks, Cold Spring Harbor Laboratories, New York, E.U.A. On a excisé un petit fragment HindIII du plasmide par digestion avec HindIII et on a religué le plasmide afin de préparer un plasmide dérivé, le YEp13 HindIII qui contient un site HindIII unique. On a fait digérer YEp13ΔHindIII purifié par HindIII et on l'a traité par la phosphatase alcaline afin d'empêcher la religation. On a récupéré l'ADN par extraction au phénol et précipitation à l'éthanol.

On a fait digérer les pRIT10671 et pRIT10672 purifiés par BamHI et on les a traités par la phosphatase alcaline afin d'empêcher la reformation du fragment pBR322. L'ADN traité a été en outre digéré par HindIII afin de libérer un fragment HindIII de 4650 pb qui contient la fusion du gène de l'HBsAg avec la région de régulation, on a extrait les échantillons par le phénol et on les a précipités à l'éthanol. On a mélangé séparément 0,4 μg de chacune des préparations d'ADN dérivées du pRIT10671 et du pRIT10672 avec 0,4 μg de YEp13 Δ HindIII digéré par HindIII et on a incubé les mélanges avec l'ADN ligase de T4.

On a utilisé une partie de chacun des mélanges ligués pour transformer les cellules compétentes de la souche MM294 de E. coli K12. On a sélectionné les transformants sur de la gélose contenant de l'ampicilline. On a isolé les colonies et on les a examinées sur base de leur contenu en plasmide selon le procédé décrit par Birnboim et al., Nucl. Acid. Res., Volume 7, 1513 (1979). Une colonie de transformant, la souche MM294 de E. coli K12 (pRIT10673) contient un plasmide, pRIT10673, qui contient le fragment HindIII en provenance du pRIT10671 et inséré sur YEp13 Δ HindIII, comme illustré dans la figure 6. Une` autre colonie de transformant, la souche MM204 de E. coli K12 (pRIT10674), contient un plasmide, le pRIT10674, qui contient le fragment HindIII en provenance de pRIT10672 et inséré sur YEp13 Δ HindIII.

Exemple 7. Transformation de la levure par pRIT10673 et pRIT10674.

On a isolé les plasmides pRIT10673 et pRIT10674 à partir des lysats éclaircis des souches MM294 (pRIT10673) et MM294 (pRIT10674) de E. coli K12 par centrifugation en gradient de densité d'un mélange CsCl-bromure d'éthidium.

A. Souche DC5 de S. cerevisiae.

La souche DC5 de S. cerevisiae (leu 2-3, leu 2-112, his 3, can 1-11) décrite par Broach et al., Gene, Volume 8, 121 (1979) a été fournie par J. Hicks, Cold Spring Harbor Laboratories, New York, E.U.A. et a été déposée le 2 juin 1982 conformément aux dispositions de la CBE et du Traité de Budapest à l'American Type Culture Collection à Rockville, Maryland, E.U.A., sous le numéro d'ordre ATCC 20630. On a cultivé les cellules de la souche DC5 et on les a préparées en vue de leur transformation selon les procédés décrits par Hinnen et al., Proc. Natl. Acad. Sci. U.S.A., Volume 75, 1929 (1978), excepté que la formation de protoplaste s'est effectuée dans 0,8 M de sorbitol, 0,03 M de β-mercaptoéthanol et 0,1 M de tampon phosphate de potassium (pH 7,5) à l'aide d'un mélange de β -glucouronidase, (concentration finale de 0,24 unité/ml) et d'arylsulfatase (concentration finale de 1,2 unité/ml). On a incubé les protoplastes de levure séparément avec 10 μg de pRIT10673 et 10 μg de pRIT10674 et on a sélectionné les transformants dans de la gélose de régénération dépourvue de leucine. On a récupéré les colonies qui croissaient dans la gélose de régénération et on les a striées sur un milieu solide contenant 0,67 % d'un milieu de base azote pour levure dépourvue en acides aminés, 2 % de glucose, 2 % de gélose et 80 $\mu$g/ml d'histidine et on les a cultivées à 30 ° C. Une colonie de la souche DC5 a été transformée par pRIT10673 et on l'appelle souche de S. cerevisiae DC5 (pRIT10673). Une autre colonie avait été transformée par pRIT10674 et on l'appelle souche de S. cerevisiae DC5 (pRIT10674). On a fait pousser des cultures des souches DC5 (pRIT10673) et DC5 (pRIT10674) dans un milieu de base azotée pour levure additionné de 80 $\mu$l/ml d'histidine jusqu'à des densités optiques de 0,33 à 1 à 620 $\mu$m. La dernière souche est utile comme témoin négatif parce qu'elle contient la séquence codante pour HBsAg fusionnée à la région de régulation dans l'orientation incorrecte. On a récupéré les cellules par centrifugation, on les a lavées avec une solution salée tamponnée au phosphate (PBS) et concentrée de 20 à 160 fois par suspension dans 5 ml de PBS additionné de 1 mM de fluorure de phénylméthyl sulfonyle (PMSF). On a brisé les cellules par deux passages à travers une cellule de pression de French à 12.000 psi (83 MPa) et on a centrifugé le lysat à 7700 x G pendant 15 minutes et ensuite à 30.000 x G pendant 30 minutes. On a récupéré les surnageants et on les a filtrés sur une membrane Millex GV.

On a testé les surnageants sur base de la présence des protéines qui réagissent avec les anticorps anti HBsAg spécifiques selon les méthodes de radioimmunodosage Ausria®. Les extraits de cellule clarifiés de la souche de S. cerevisiae DC5 (pRIT10673) préparés de cette manière ont donné des réactions positives dans ce radioimmunodosage même lorsqu'ils ont été testés à des dilutions de 16 à 256 fois dans le PBS. Au contraire, en ce qui concerne la présence des protéines réagissant avec les anticorps anti-HBsAg, les extraits de la souche DC5 (pRIT10674) ont été négatifs dans cet essai.

B. Souche lc1697d de S. cerevisiae.

La souche lc1697d de S. cerevisiae a été déposée le 2 juin 1982 conformément aux dispositions de la CBE et du Traité de Budapest, à l'American Type Culture Collection sous le numéro d'ordre ATCC 20631. En utilisant le procédé décrit ci-dessus, on a transformé la souche bradytrophe pour l'arginine lc1697d (argJ $^+$, leu 2-1) avec pRIT10673 et pRIT10674. Une colonie leucine indépendante de la souche bradytrophe a été transformée par pRIT10673 et on l'appelle souche de S. cerevisiae lc1697d (pRIT10673). Une autre colonie leucine indépendante que l'on a transformée par pRIT10674 est appelée souche de S. cerevisiae lc1697d (pRIT10674). On a fait pousser les cultures des souches lc1697d (pRIT10673) et lc1697d (pRIT10674) dans un milieu de base azotée pour levure additionné de 20 $\mu$g/ml d'arginine. On a récupéré les cellules et on a préparé les extraits des cellules comme décrit ci-dessus. Les extraits de cellules clarifiées de la souche lc1697d (pRIT10673) ont donné des résultats positifs dans le radioimmunodosage Ausria® à des dilutions allant jusqu'à 1/2048 tandis que les extraits de la souche lc1697d (pRIT10674) se sont avérés uniformément négatifs dans cet essai.

A partir de ces résultats, on conclut que les cellules de levure des souches DC5 et lc697d transformées avec pRIT10673 synthétisent spécifiquement HBsAg sous la forme d'une protéine de fusion possédant les déterminants de HBsAg.

Exemple 8. Immunisation des lapins avec HBsAg au départ de la souche de S. cerevisiae lc1697d (pRIT10673).

On a cultivé la souche de S. cerevisiae lc1697 (pRIT10673) jusqu'à une densité optique de 0,60 à 620 $\mu$m et on l'a recueillie par centrifugation. On a remis en suspension les cellules concentrées 40 fois dans du PBS additionné de 1 mM de PMSF. On a préparé un extrait clarifié de cellules comme décrit dans l'exemple 7. On a préparé un extrait clarifié de cellule de la souche lc1697d (pRIT10674). Ces extraits ont été utilisés pour immuniser des lapins. Un premier groupe de 6 lapins a reçu des injections parentérales de 1 ml d'extrait de la souche lc1697d ((pRIT10673) mélangé avec 1 ml d'adjuvant complet de Freund aux jours 0, 9, 15, 30 et 37. Ces mêmes jours, un second groupe de trois lapins a reçu des injections parentérales de 1 ml d'extrait de la souche lc1697 (pRIT10674) mélangé avec 1 ml d'adjuvant complet de Freund. On a recueilli les sérums des deux groupes les jours 0 (préimmun), 23, 51 et 65 et ceux du premier groupe, au jour 44 et on les a testés pour leur présence en anticorps anti-HBsAg en utilisant le radioimmunodosage Ausab®. Les résultats de ces essais, qui sont fournis dans le tableau 1, indiquent que quatre lapins sur six ayant reçu des injections d'extrait de la souche lc1697 (pRIT10673) produisaient des anticorps dirigés contre l'HBsAg. Aucun des trois lapins témoins ayant reçu des injections d'extrait de la souche lc1697d (pRIT10674) ne manifestait de signe de production d'anticorps anti-HBsAg. A partir de ces résultats, on a conclu que les extraits de la souche lc1697d (pRIT10673) contiennent HBsAg qui peut être utilisé sans effets secondaires graves dans un vaccin pour stimuler la protection contre l'infection à HBV chez les humains.

TABLEAU 1

| PRODUCTION D'ANTICORPS ANTI-HBsAg PAR LES LAPINS IMMUNISES AVEC DES EXTRAITS ACELLULAIRES DES SOUCHES DE S. CEREVISIAE lc1697d (pRIT10673) ET lc1697d (pRIT10674). | | | | | | |
|---|---|---|---|---|---|---|
| SOURCE D'EXTRAIT | LAPIN ANTI-HBsAg | | TITRE DES SERUMS PAR DOSAGE AUSAB® ( a) | | | |
| | NUMERO | JOUR 0 | JOUR 23 | JOUR 44 | JOUR 51 | JOUR 65 |
| souche 1cl697d -(pRIT10673) | 1 | nég | nég | 1/16 | 1/16 | 1/64 |
| | 2 | nég | 1/1024 | 1/8192 | 1/8192 | 1/4096 |
| | | | | | | 1/83 |
| | 3 | nég | 1/64 | 1/16 | 1/16 | 1/16 |
| | 4 | nég | 1/64 | 1/2048 | 1/1024 | 1/2048 |
| | 5 | nég | 1/1024 | 1/256 | 1/1024 | 1/2048 |
| | 6 | nég | 1/16 | 1/256 | 1/1024 | 1/2048 |
| souche 1cl697d -(pRIT10674 | 7 | nég | nég | --- | nég | --- |
| | 8 | nég | 1/1 | --- | nég | nég |
| | 9 | nég | nég | --- | nég | nég |

aDilution la plus élevée de sérum donnant un résultat positif dans le dosage Ausab®

**Revendications**

**Revendications pour les Etats contractants suivants : BE, CH, DE, FR, GB, IT, LI, LU, NL, SE**

1. Molécule d'ADN recombinant comprenant une séquence nucléotidique qui code pour HBsAg et une région de régulation qui peut effectuer la transcription dans la levure, ladite séquence étant exprimée en un polypeptide conservant l'immunogénicité du HBsAg et comprenant le polypeptide HBsAg de 226 acides aminés et une extension polypeptidique à son extrémité N-terminale caractérisée en ce que l'extension est un polypeptide dont au moins une partie ne dérive pas de la région preS, dans laquelle la séquence comprend une séquence qui code pour une extension comprenant à la fois un polypeptide qui dérive de la région preS et un polypeptide qui n'en dérive pas, et dans laquelle la séquence comprend une séquence qui code pour une extension qui dérive de la région preS est formée par une séquence contenant jusqu'aux 42 acides aminés N-terminaux qui précèdent naturellement le polypeptide HBsAg de 226 acides aminés.

2. Molécule d'ADN recombinant suivant la revendication 1 dans laquelle la partie de l'extension qui dérive de la région preS est formée par les 42 acides aminés N-terminaux qui précèdent naturellement le polypeptide HBsAg de 226 acides aminés.

3. Molécule d'ADN recombinant suivant la revendication 2 dans laquelle la région qui n'en dérive pas est la région codant pour les 18 acides aminés N-terminaux de l'ornithine carbamoyltransférase.

4. Levure transformée par un plasmide contenant une molécule recombinante suivant l'une quelconque des revendications 1 à 3.

5. Levure suivant la revendication 4, caractérisée en ce qu'elle est S. cerevisiae.

6. Procédé pour la production d'un polypeptide, caractérisé en ce qu'il comprend la culture d'une levure suivant la revendication 4 ou 5 et que l'on récolte le polypeptide produit.

7. Dérivé immunogène de HBsAg comprenant le polypeptide HBsAg de 226 acides aminés et une extension polypeptidique à son extrémité N-terminale, caractérisé en ce que l'extension comprend un polypeptide dont au moins une partie ne dérive pas de la région preS, et caractérisé en ce que l'extension comprend à la fois un polypeptide qui dérive de la région preS et un polypeptide qui n'en dérive pas, dans laquelle la partie d'extension qui dérive de la région preS est formée par une séquence contenant jusqu'aux 42 acides aminés qui précèdent naturellement le polypeptide HBsAg de 226 acides aminés.

**8.** Dérivé immunogène de HBsAg suivant la revendication 7, caractérisé en ce que la partie de l'extension qui dérive de la région preS est formée par les 42 acides aminés qui précèdent naturellement le polypeptide HBsAg de 226 acides aminés.

**9.** Dérivé immunogène de HBsAg suivant la revendication 8, caractérisé en ce que la partie de l'extension qui ne dérive pas de la région preS est formée par les 18 acides aminés N'terminaux de l'ornithine carbamoyltransférase.

**10.** Vaccin caractérisé en ce qu'il comprend un dérivé immunogène de HBsAg suivant l'une quelconque des revendications 7 à 9 et un excipient convenable.

**Revendications pour l'Etat contractant suivant : AT**

**1.** Levure transformée par un plasmide contenant une molécule d'ADN recombinant comprenant une séquence nucléotidique qui code pour HBsAg et une région de régulation qui peut effectuer la transcription dans la levure, la séquence étant exprimée en un polypeptide conservant l'immunogenicité du HBsAg et comprenant le polypeptide HBsAg de 226 acides aminés et une extension polypeptidique à son extrémité N-terminale, caractérisée en ce que l'extension du polypeptide exprimé est un polypeptide dont au moins une partie ne dérive pas de la région preS, dans laquelle l'extension du polypeptide exprimé comprend à la fois un polypeptide qui dérive de la région preS et un polypeptide qui n'en dérive pas, et dans laquelle la partie de l'extension qui dérive de la région preS est formée par une séquence contenant jusqu'aux 42 acides aminés N-terminaux qui précèdent le polypeptide HBsAg de 226 acides aminés.

**2.** Levure suivant la revendication 1 dans laquelle la partie de l'extension qui dérive de la région preS est formée par les 42 acides aminés N-terminaux qui précèdent le polypeptide HBsAg de 226 acides aminés.

**3.** Levure suivant l'une quelconque des revendications 1 à 2 caractérisé en ce qu'il est S. cerevisiae.

**4.** Procédé pour la production d'un polypeptide, caractérisé en ce qu'il comprend la culture d'une levure suivant l'une quelconque des revendications 1 à 3 et que l'on recolte le polypeptide produit.

**5.** Procédé pour la préparation d'un vaccin contenant HBsAg recombinant, caractérisé en ce qu'on utilise un polypeptide conservant l'immunogenicité du HBsAg et comprenant le polypeptide HBsAg de 226 acides aminés avec une extension polypeptidique à son extrémité N-terminale et un excipient convenable, et caractérisé par le fait que l'extension comprend un polypeptide dont au moins une partie ne dérive pas de la région preS, dans laquelle l'extension comprend à la fois un polypeptide qui dérive de la région preS et un polypeptide qui n'en dérive pas et dans laquelle la partie de l'extension qui dérive de la région preS est formée par une séquence contenant jusqu'aux 42 acides aminés qui précèdent naturellement le polypeptide HBsAg de 226 acides aminés.

**6.** Procédé suivant la revendication 5, caractérisé en ce que la partie de l'extension qui dérive de la région preS est formée par les 42 acides aminés qui précèdent naturellement le polypeptide HBsAg de 226 acides aminés.

**7.** Procédé suivant la revendication 6, caractérisé en ce que la partie de l'extension qui ne dérive pas de la région preS est formée par les 18 acides aminés N-terminaux de l'ornithine carbamoyltransférase

**Claims**

**Claims for the following Contracting States : BE, CH, DE, FR, GB, IT, LI, LU, NL, SE**

1. Recombinant DNA molecule comprising a nucleotide sequence which codes for HBsAg and a regulatory region which can effect transcription in yeast, the said sequence being expressed as a polypeptide that retains the immunogenicity of HBsAg and comprises the HBsAg polypeptide of 226 amino acids and a polypeptide extension at its N-terminal end, characterised in that the extension is a polypeptide at least a portion of which is not derived from the pre-S region, in which the sequence comprises a sequence which codes for an extension comprising both a polypeptide which is derived from the pre-S region and a polypeptide which is not derived therefrom, and in which the sequence comprises a sequence which codes for an extension which is derived from the pre-S region and is composed of a sequence containing up to the 42 N-terminal amino acids which naturally precede the HBsAg polypeptide of 226 amino acids.

2. Recombinant DNA molecule according to Claim 1, in which the portion of the extension which is derived from the pre-S region is composed of the 42 N-terminal acids which naturally precede the HBsAg polypeptide of 226 amino acids.

3. Recombinant DNA molecule according to Claim 2, in which the region which is not derived therefrom is the region coding for the 18 N-terminal amino acids of ornithine carbamoyltransferase.

4. Yeast transformed by a plasmid containing a recombinant molecule according to any one of Claims 1 to 3.

5. Yeast according to Claim 4, characterised in that it is S. cerevisiae.

6. Method for the production of a polypeptide, characterised in that it comprises the culture of a yeast according to Claim 4 or 5 and in that the peptide produced is harvested.

7. Immunogenic derivative of HBsAg comprising the HBsAg polypeptide of 226 amino acids and a polypeptide extension at its N-terminal end, characterised in that the extension comprises a polypeptide at least a portion of which is not derived from the pre-S region, and characterised in that the extension comprises both a polypeptide which is derived from the pre-S region and a polypeptide which is not derived therefrom, in which the portion of extension which is derived from the pre-S region is composed of a sequence containing up to the 42 amino acids which naturally precede the HBsAg polypeptide of 226 amino acids.

8. Immunogenic derivative of HBsAg according to Claim 7, characterised in that the portion of the extension which is derived from the pre-S region is composed of the 42 amino acids which naturally precede the HBsAg polypeptide of 226 amino acids.

9. Immunogenic derivative of HBsAg according to Claim 8, characterised in that the portion of the extension which is not derived from the pre-S region is composed of the 18 N-terminal amino acids of ornithine carbamoyltransferase.

10. Vaccine, characterised in that it comprises an immunogenic derivative of HBsAg according to any one of Claims 7 to 9 and a suitable excipient.

**Claims for the following Contracting State : AT**

1. Yeast transformed by a plasmid containing a recombinant DNA molecule comprising a nucleotide sequence which codes for HBsAg and a regulatory region which can effect transcription in yeast, the sequence being expressed as a polypeptide that retains the immunogenicity of HBsAg and comprises the HBsAg polypeptide of 226 amino acids and a polypeptide extension at its N-terminal end, characterised in that the extension of the polypeptide expressed is a polypeptide at least a portion of which is not derived from the pre-S region, in which the extension of the polypeptide expressed comprises both a polypeptide which is derived from the pre-S region and a polypeptide which is not derived therefrom, and in which the portion of the extension which is derived from the pre-S region is composed of a sequence containing up to the 42 N-terminal amino acids which precede the HBsAg polypeptide of 226 amino acids.

2. Yeast according to Claim 1, in which the portion of the extension which is derived from the pre-S region is composed of the 42 N-terminal amino acids which precede the HBsAg polypeptide of 226 amino acids.

3. Yeast according to either of Claims 1 and 2, characterised in that it is S. cerevisiae.

4. Method for the production of a polypeptide, characterized in that it comprises the culture of a yeast according to any one of Claims 1 to 3 and in that the polypeptide produced is harvested.

5. Method for the preparation of a vaccine containing recombinant HBsAg, characterized in that a polypeptide that retains the immunogenicity of HBsAg and comprises the HBsAg polypeptide of 226 amino acids with a polypeptide extension at its N-terminal end and a suitable excipient are used, and characterised in that the extension comprises a polypeptide at least a portion of which is not derived from the pre-S region, in which the extension comprises both a polypeptide which is derived from the pre-S region and a polypeptide which is not derived therefrom, and in which the portion of the extension which is derived from the pre-S region is composed of a sequence containing up to the 42 amino acids which naturally precede the HBsAg polypeptide of 226 amino acids.

6. Method according to Claim 5, characterised in that the portion of the extension which is derived from the pre-S region is composed of the 42 amino acids which naturally precede the HBsAg polypeptide of 226 amino acids.

7. Method according to Claim 6, characterised in that the portion of the extension which is not derived from the pre-S region is composed of the 18 N-terminal amino acids of ornithine carbamoyltransferase.

**Patentansprüche**

**Patentansprüche für folgende Vertragsstaaten : BE, CH, DE, FR, GB, IT, LI, LU, NL, SE**

1. Rekombinantes DNA-Molekül, umfassend eine HBsAg codierende Nucleotidsequenz und einen Regulationsbereich, der die Transkription in Hefe bewirken kann, wobei die Sequenz ein Polypeptid exprimiert, das die Immunogenität von HBsAg bewahrt und das Polypeptid HBsAg mit 226 Aminosäuren sowie ein erweiterndes Polypeptid am N-terminalen Ende umfaßt, dadurch gekennzeichnet, daß die Erweiterung ein Polypeptid ist, von dem mindestens ein Teil nicht von dem Bereich preS stammt, wobei die Sequenz eine Sequenz umfaßt, die eine Erweiterung codiert, welche gleichzeitig ein Polypeptid, das von der Region preS stammt und ein Polypeptid, das nicht von dieser Region stammt, umfaßt, und wobei die Sequenz, die eine Sequenz umfaßt, die eine Erweiterung codiert, welche von dem Bereich preS stammt, durch eine Sequenz gebildet wird, die bis zu 42 N-terminale Aminosäuren umfaßt, die natürlicherweise dem Polypeptid HBsAg mit 226 Aminosäuren vorausgehen.

2. Rekombinantes DNA-Molekül nach Anspruch 1, wobei der Erweiterungsteil, welcher von dem Bereich preS stammt, von den 42 N-terminalen Aminosäuren gebildet wird, die natürlicherweise dem Polypeptid HBsAg mit 226 Aminosäuren vorausgehen.

3. Rekombinantes DNA-Molekül nach Anspruch 2, wobei der Bereich, der nicht davon abstammt, der Bereich ist, der die 18 N-terminalen Aminosäuren von Ornithincarbamoyltransferase codiert.

4. Mit einem Plasmid transformierte Hefe, wobei das Plasmid ein rekombinantes Molekül gemäß einem der Ansprüche 1 bis 3 enthält.

5. Hefe nach Anspruch 4, dadurch gekennzeichnet, daß sie S. cerevisiae ist.

6. Verfahren zur Herstellung eines Polypeptids, dadurch gekennzeichnet, daß es die Züchtung einer Hefe nach den Ansprüchen 4 oder 5 und die Gewinnung des erhaltenen Polypeptids umfaßt.

7. Immunogenes Derivat von HBsAg, umfassend das Polypeptid HBsAg mit 226 Aminosäuren und eine Polypeptiderweiterung am N-terminalen Ende, dadurch gekennzeichnet, daß die Erweiterung ein Polypeptid umfaßt, von dem mindestens ein Teil nicht von dem Bereich preS stammt, und dadurch gekennzeichnet, daß die Erweiterung gleichzeitig ein Polypeptid umfaßt, das von dem Bereich preS stammt, und ein Polypeptid, das nicht davon stammt, wobei der Erweiterungsteil, der von dem Bereich preS stammt, von einer Sequenz gebildet wird, die bis zu 42 Aminosäuren umfaßt, die natürlicherweise dem Polypeptid HBsAg mit 226 Aminosäuren vorausgehen.

8. Immunogenes Derivat von HBsAg nach Anspruch 7, dadurch gekennzeichnet, daß der Erweiterungsteil, der von dem Bereich preS stammt, durch die 42 Aminosäuren gebildet wird, die natürlicherweise dem Polypeptid HBsAg mit 226 Aminosäuren vorausgehen.

9. Immunogenes Derivat von HBsAg nach Anspruch 8, dadurch gekennzeichnet, daß der Erweiterungsteil, der nicht von dem Bereich preS stammt, von den 18 N-terminalen Aminosäuren von Ornithincarbamoyltransferase gebildet wird.

10. Impfstoff, dadurch gekennzeichnet, daß er ein immunogenes Derivat von HBsAg nach einem der Ansprüche 7 bis 9 und einen verträglichen Träger umfaßt.

**Patentansprüche für folgenden Vertragsstaat : AT**

1. Mit einem Plasmid transformierte Hefe, wobei das Plasmid ein rekombinantes Molekül enthält, umfassend eine HBsAg codierende Nucleotidsequenz und einen Regulationsbereich, der die Transkription in Hefe bewirken kann, wobei die Sequenz ein Polypeptid exprimiert, das die Immunogenität von HBsAg bewahrt und das Polypeptid HBsAg mit 226 Aminosäuren sowie ein erweiterndes Polypeptid am N-terminalen Ende umfaßt, dadurch gekennzeichnet, daß die Erweiterung des exprimierten Polypeptids ein Polypeptid ist, von dem mindestens ein Teil nicht von dem Bereich preS stammt, wobei die Erweiterung des exprimierten Polypeptids gleichzeitig ein Polypeptid, das von der Region preS stammt und ein Polypeptid, das nicht von dieser Region stammt, umfaßt, und wobei der Erweiterungsteil, der von dem Bereich preS stammt, von einer Sequenz gebildet wird, die bis zu 42 N-terminale Aminosäuren umfaßt, die dem Polypeptid HBsAg mit 226 Aminosäuren vorausgehen.

2. Hefe nach Anspruch 1, wobei der Erweiterungsteil, welcher von dem Bereich preS stammt, von den 42 N-terminalen Aminosäuren gebildet wird, die dem Polypeptid HBsAg mit 226 Aminosäuren vorausgehen.

3. Hefe nach einem der Ansprüche 1 bis 2, dadurch gekennzeichnet, daß sie S. cerevisiae ist.

4. Verfahren zur Herstellung eines Polypeptids, dadurch gekennzeichnet, daß es die Züchtung einer Hefe nach einem der Ansprüche 1 bis 3 und die Gewinnung des erhaltenen Polypeptids umfaßt.

5. Verfahren zur Herstellung eines rekombinanten HbsAg enthaltenden Impfstoffs, dadurch gekennzeichnet, daß man ein Polypeptid, das die Immunogenität von HbsAg bewahrt und das Polypeptid HBsAg mit 226 Aminosäuren und einer Polypeptiderweiterung am N-terminalen Ende umfaßt, und einen verträglichen Träger verwendet, und dadurch gekennzeichnet, daß die Erweiterung ein Polypeptid umfaßt, von dem mindestens ein Teil nicht von dem Bereich preS stammt, wobei die Erweiterung gleichzeitig ein Polypeptid umfaßt, das von dem Bereich preS stammt, und ein Polypeptid, das nicht davon stammt, und wobei der Erweiterungsteil, der von dem Bereich preS stammt, von einer Sequenz gebildet wird, die bis zu 42 Aminosäuren umfaßt, die natürlicherweise dem Polypeptid HBsAg mit 226 Aminosäuren vorausgehen.

6. Verfahren nach Anspruch 5, dadurch gekennzeichnet, daß der Erweiterungsteil, der von dem Bereich preS stammt, durch die 42 Aminosäuren gebildet wird, die natürlicherweise dem Polypeptid HBsAg mit 226 Aminosäuren vorausgehen.

7. Verfahren nach Anspruch 6, dadurch gekennzeichnet, daß der Erweiterungsteil, der nicht von dem Bereich preS stammt, von den 18 N-terminalen Aminosäuren von Ornithincarbamoyltransferase gebildet wird.

Figure 1

EcoRI
Pst I
BamHI
Hinc II
Xba I

Hinc II

BamHI
Ava I

Hinc II

Bgl II
Taq I

Bgl II
Ava I
Hinc II

Hinc II

Hinc II
EcoRI

Hinc II

Ava I

Hinc II
tet

BamHI

Hind III
Xba I

pRIT10616

7450 bp

pACYC184

HBV DNA

Figure 2

Figure 3

```
TCTAAAGGCA        GTTTATTCCT        TGTATGTCCT

TTAAGTACAG        TTAATAACGA        GCAATTTTTT
    RsaI

TTTTTTTTTT        TAGCCATCTA        CCCATCAACT

TGTACACTCG        TTACC[ATG]TC      AACCACAGCA
  RsaI                     HincIII

TCCACGCCTT        CATCTTTACG        TCATTTGATT

TCTATAAAAG        ATCTTTCTGA        TGAAGAATTC
        BglII                             EcoRI

AGAATCTTAG        TACAAAGAGC        TCAACATTTC
```

## Figure 4

EP 0 106 828 B1

Figure 5